(19) Europäisches Patentamt
European Patent Office
Office européen des brevets

(11) **EP 1 857 118 A1**

(12) **EUROPEAN PATENT APPLICATION**
published in accordance with Art. 158(3) EPC

(43) Date of publication:
**21.11.2007 Bulletin 2007/47**

(51) Int Cl.:
*A61K 45/00* [(2006.01)]    *A61K 31/155* [(2006.01)]
*A61K 31/427* [(2006.01)]    *A61P 3/10* [(2006.01)]

(21) Application number: **05814764.6**

(22) Date of filing: **12.12.2005**

(86) International application number:
**PCT/JP2005/022739**

(87) International publication number:
**WO 2006/064744 (22.06.2006 Gazette 2006/25)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **13.12.2004 JP 2004359587**

(71) Applicant: **Daiichi Sankyo Company, Limited Tokyo 103-8426 (JP)**

(72) Inventors:
• **YOSHIDA, Taishi**
**Shinagawa-ku**
**Tokyo 140-8710 (JP)**

• **OKUNO, Akira**
**1-2-58, Hiromachi**
**Shinagawa-ku**
**Tokyo 140-8710 (JP)**

(74) Representative: **Gibson, Christian John Robert Marks & Clerk**
**90 Long Acre**
**London WC2E 9RA (GB)**

(54) **MEDICINAL COMPOSITION FOR TREATING DIABETES**

(57)    To provide a therapeutic procedure for diabetes mellitus with an excellent efficacy and high safety by possibly reducing side effects.

To administer a pharmaceutical composition comprising an FBPase inhibitor at a specified timing.

(Fig. 2)

EP 1 857 118 A1

**Description**

[Technical field]

The present invention relates to a therapeutic agent containing an FBPase inhibitor as an active ingredient, which is a highly efficacious agent against diabetes mellitus, hyperglycemia, impaired glucose tolerance, obesity, and diabetic complications (preferably diabetes mellitus) and furthermore may reduce adverse events, and to methods for its administration.

In addition, the present invention relates to a therapeutic agent containing an FBPase inhibitor and other antidiabetic agents as active ingredients, which is a highly efficacious agent for diabetes mellitus, hyperglycemia, impaired glucose tolerance, obesity, and diabetic complications (preferably diabetes mellitus) and furthermore may reduce adverse events, and to methods for its administration.

[Background art]

FBPase inhibitors are known to reduce blood glucose levels by inhibiting gluconeogenesis in the liver. Thus it is considered that FBPase inhibitors are effective as therapeutic agents against diabetes mellitus, hyperglycemia, impaired glucose tolerance, obesity, and diabetic complications (for example, retinopathy, cataract, nephropathy, peripheral nerve injury, and the like) (for example, see Patent Literatures 1 to 3).

On the other hand, since biguanide preparations exhibit lowering activity on blood glucose levels without weight gain, these preparations are widely used as therapeutic agents for diabetes mellitus in Europe and the USA. Metformin is a currently available biguanide preparation. This agent is administered to patients with diabetes mellitus twice daily. However, it has been known that when biguanide preparations such as metformin and the like are clinically used, they possibly elicit adverse events such as gastrointestinal disorders (for example, nausea, dyspepsia, and the like), and, in rare cases, lactic acidosis could be elicited by accumulation of lactic acid. Thus adequate attentions should be paid when biguanide preparations are clinically used.

In addition, it is well known that regimens containing two or more antidiabetic agents are often prescribed to patients with diabetes mellitus. However, in the conventional combination administration methods, the other antidiabetic agent(s) are generally added to the currently used agents, and only some additive effect is expected. Consequently, there is now a need to develop the most appropriate administration methods of combined antidiabetic agents, that is, efficacious administration methods by which each agent having different modes of action can exert its own merits to the maximum extent and by which at the same time the weak points (side effects and the like) can be suppressed as much as possible.

There have been a number of literatures disclosing lowering effects on blood glucose levels of combined administration of an FBPase inhibitor and other anti-diabetic agents (for example, see Patent Literature 4). However, there is neither description nor suggestion of the specified administration methods discovered by the present invention in these literatures.

[Patent Literature 1] International publication number WO 00/14095
[Patent Literature 2] International publication number WO 01/47935
[Patent Literature 3] International publicatiom number WO 01/66553
[Patent Literature 4] International publication number WO 02/03978

[Disclosure of the invention]

[Object of the invention]

The present inventors have diligently conducted research to discover therapeutic procedures with high safety for patients with diabetic mellitus, by exhibiting excellent therapeutic efficacies as well as suppressing side effects (for example, gastrointestinal disorders) and have discovered that high therapeutic efficacies and remarkable suppression of side effects could be obtained by administration of agents containing an FBPase inhibitor at a specified administration timing. Thus the present inventors have completed the present invention.

Furthermore, the present inventors have discovered that combined administration of an FBPase inhibitor and other anti-diabetic agent(s) (preferably a biguanide preparation) at specified administration timings could exert high therapeutic efficacies in terms of blood glucose lowering effects and could suppress side effects remarkably, and consequently, the present inventors have completed the present invention.

[Means to achieve the object]

The present invention relates to

(1) a pharmaceutical composition comprising an FBPase inhibitor for prophylaxis or treatment of diabetes mellitus, which is characterized by augmentation of the prophylactic or therapeutic effects on diabetes mellitus when the pharmaceutical composition is administered at an early time of the longest meal interval,

(2) a pharmaceutical composition comprising an FBPase inhibitor for prophylaxis or treatment of diabetes mellitus, which is characterized by augmentation of the prophylactic or therapeutic effects on diabetes mellitus when the pharmaceutical composition is orally administered once daily at an early time of the longest meal interval,

(3) a pharmaceutical composition according to (1) or (2) described above, which is administered at a certain time in the period from after dinner to bedtime,

(4) a pharmaceutical composition according to any one selected from (1) to (3) described above, which is administered at a certain time after dinner,

(5) a pharmaceutical composition according to any one selected from (1) to (4) described above, in which an FBPase inhibitor is contained in an amount of from 1 mg to 400 mg,

(6) a pharmaceutical composition according to any one selected from (1) to (4) described above, in which an FBPase inhibitor is contained in an amount of from 10 mg to 200 mg,

(7) a kit of a pharmaceutical composition for prophylaxis or treatment of diabetes mellitus, in which at least a single unit of an FBPase inhibitor containing an amount of from 1 mg to 400 mg as a single dose is contained with an indication to take the kit orally once daily at an early time in the period from after dinner to bedtime,

(8) a method of treatment for diabetes mellitus, which is characterized by once daily oral administration of a pharmaceutical composition comprising an FBPase inhibitor as an active ingredient, at an early time of the longest meal interval,

(9) a kit of a pharmaceutical composition for the treatment of diabetes mellitus comprising two different active ingredients to be separately administered at an appropriate time interval, which contains (a) a pharmaceutical composition comprising a biguanide preparation as the first component of the active ingredients, and (b) a pharmaceutical composition comprising an FBPase inhibitor as the second component of the active ingredients,

(10) a kit of a pharmaceutical composition for the treatment of diabetes mellitus comprising two different active ingredients to be separately administered at an appropriate time interval to prevent adverse events, which contains (a) a pharmaceutical composition comprising a biguanide preparation as the first component of the active ingredients, and (b) a pharmaceutical composition comprising an FBPase inhibitor as the second component of the active ingredients,

(11) a kit of a pharmaceutical composition for the treatment of diabetes mellitus comprising two different active ingredients to be separately administered at an appropriate time interval to prevent adverse events by reducing the dose of a biguanide preparation, which contains (a) a pharmaceutical composition comprising a biguanide preparation as the first component of the active ingredients, and (b) a pharmaceutical composition comprising an FBPase inhibitor as the second component of the active ingredients,

(12) a kit of a pharmaceutical composition for the treatment of diabetes mellitus according to any one selected from (8) to (11) described above, wherein it is clearly indicated that (a) the first component of the active ingredients is to be administered at a time when the target patients' glucose uptake into target tissues of the treatment is increased, and (b) the second component of the active ingredients is to be administered at a time when the patients' gluconeogenesis targeted by the treatment is predominantly facilitated,

(13) a kit of a pharmaceutical composition for the treatment of diabetes mellitus according to any one selected from (8) to (11) described above, wherein it is clearly indicated that the second component of the active ingredients is to be administered to the target patients at a time in the period from after dinner to bedtime,

(14) a kit of a pharmaceutical composition for the treatment of diabetes mellitus according to any one selected from (8) to (11) described above, wherein it is clearly indicated that (a) the first component of the active ingredients is to be administered to the target patients at a time in the period from 4: 00 to 12: 00, while (b) the second component of the active ingredients is to be administered at a time in the period from 17:00 to 1:00,

(15) a kit of a pharmaceutical composition for the treatment of diabetes mellitus according to any one selected from (10) to (14) described above, wherein it is indicated that adverse events of the pharmaceutical composition are gastrointestinal disorders,

(16) a kit of a pharmaceutical composition for the treatment of diabetes mellitus according to any one selected from (10) to (15) described above, wherein it is indicated that an adverse event of the pharmaceutical composition is an increase of lactic acid,

(17) a kit of a pharmaceutical composition for the treatment of diabetes mellitus according to any one selected from (8) to (16) described above, wherein the kit is packed for oral administration,

(18) a kit of a pharmaceutical composition for the treatment of diabetes mellitus according to any one selected from (8) to (17), wherein the biguanide preparation is metformin, phenformin, or buformin,

(19) a kit of a pharmaceutical composition for the treatment of diabetes mellitus according to any one selected from (8) to (17) described above, wherein the biguanide preparation is metformin,

(20) a kit of a pharmaceutical composition for the treatment of diabetes mellitus according to any one selected from (8) to (19) described above, wherein the FBPase inhibitor is an agent that inhibits human FBPase activity,

(21) a kit of a pharmaceutical composition for the treatment of diabetes mellitus according to any one selected from (8) to (19) described above, wherein the FBPase inhibitor is 2-amino-5-isobutyl-4-{2-[5-(N,N'-bis((S)-1-ethoxycarbonyl)e thyl)phosphonamido]furanyl}thiazole or a pharmacologically acceptable salt thereof,

(22) use of a biguanide preparation and an FBPase inhibitor for the manufacture of a kit of a pharmaceutical composition for the treatment of diabetes mellitus comprising two different active ingredients, (a) a pharmaceutical composition comprising a biguanide preparation as the first component of the active ingredients and (b) a pharmaceutical composition comprising an FBPase inhibitor as the second component of the active ingredients, to be separately administered for treatment of diabetes mellitus at an appropriate time interval,

(23) a therapeutic agent for diabetes mellitus, which is characterized by administering an agent containing a biguanide preparation as an active ingredient and an agent containing an FBPase inhibitor as an active ingredient separately at an appropriate time interval,

(24) a combination of therapeutic agents for diabetes mellitus used in relation to each other, which is characterized by administering (a) a pharmaceutical composition comprising a biguanide preparation as an active component and (b) a pharmaceutical composition comprising an FBPase inhibitor as an active ingredient separately at an appropriate time interval,

(25) a method of treatment for diabetes mellitus, which is characterized by separate administration of (a) a pharmaceutical composition comprising a biguanide preparation as an active ingredient, and (b) a pharmaceutical composition comprising an FBPase inhibitor as an active ingredient at an appropriate time interval, and

(26) a method of treatment for diabetes mellitus, which is characterized by administering (a) a pharmaceutical composition comprising a biguanide preparation as an active ingredient and (b) a pharmaceutical composition comprising an FBPase inhibitor as an active ingredient separately at an appropriate time interval to reduce adverse events caused by the biguanide preparation.

In the present invention, "FBPase inhibitors" are not particularly restricted provided that they inhibit FBPase activity, and they are, for example, phosphoramide ester compounds containing the phosphoramide ester structure as a prodrug moiety which are disclosed in International publication number WO 01/47935 pamphlet, or compounds disclosed in International publication number WO 00/14095pamphlet, Journal of Medicinal Chemistry, Vol. 45, 3865-3877, 2002, and Bioorganic & Medicinal Chemistry Letters, Vol. 11, 17-21, 2001, preferably phosphoramide ester compounds, and particularly preferably 2-amino-5-isobutyl-4-{2-[5-(N,N'-bis((S)-1-ethoxycarbonyl)ethyl) phosphonamido]furanyl}thiazole and pharmacologically acceptable salts thereof.

In the present invention, "therapeutic agents for diabetes mellitus" are not particularly restricted provided that they are generally prescribed to patients with diabetes mellitus, and they are, for example, insulin preparations, biguanide preparations, insulin secretion enhancers (SU preparations and the like), digesting enzyme inhibitors ($\alpha$-glucosidase inhibitor and the like), and insulin sensitizers, and the like.

In the present invention, "biguanide preparations" are not particularly restricted provided that they exhibit acceleration of anaerobic glycolysis, potentiation of insulin action at peripheral sites, suppression of glucose uptake from the intestinal canal, and inhibition of gluconeogenesis in the liver, and the like, and they are, for example, 1,1-dimethylbiguanide monohydrochloride (generic name: metformin), phenformin, buformin, and the like, and particularly preferably metformin.

The administration route of therapeutic agents against diabetes mellitus employed in the present invention is generally oral administration. The dosage forms are not particularly restricted provided that they are prepared using conventional formulation techniques, and they are powder, granules, tablets, and capsules.

These preparations are prepared by conventionally known methods using known additive agents in the formulation field of remedies such as excipients, binders, disintegrants, lubricants, dissolution agents, flavors, coating agents, and the like.

For example, when tablets are prepared, inactive ingredients conventionally employed in this field may be widely used. For instance, they may include excipients such as lactose, sucrose, sodium chloride, glucose, urea, starch, calcium carbonate, kaolin, crystalline cellulose, silicic acid, and the like; binders such as water, ethanol, propanol, syrup, glucose solution, starch solution, gelatin solution, carboxymethylcellulose, shellac, methylcellulose, potassium phosphate, polyvinylpyrrolidone, and the like; disintegrants such as dried starch, sodium alginate, agar powder, laminaran powder, sodium hydrogen carbonate, calcium carbonate, polyoxyethylene sorbitan fatty acid esters, sodium lauryl sulfate, stearic acid monoglyceride, starch, lactose, and the like; disintegration inhibitors such as sucrose, stearin, cacao butter, hydrogenated oil, and the like; absorption enhancers such as quaternary ammonium bases, sodium lauryl sulfate, and the like; humectants such as glycerin, starch, and the like; adsorbents such as starch, lactose, kaolin, bentonite, colloidal silicic acid, and the like; and lubricants such as purified talc, stearates, boric acid powder, polyethylene glycol, and the like. In addition, tablets may be conventionally coated when required, for example, they may be prepared as sugar-coated tablets, gelatin-coated tablets, enteric-coated tablets, film-coated tablets, double-coated tablets, and multi-layer tablets.

When pills are prepared, well-known inactive ingredients that are conventionally employed in this field may be widely used. For instance, excipients such as glucose, lactose, starch, cacao butter, hardening vegetable oil, kaolin, talc, and the like; binders such as gum arabic acacia powder, tragacanth powder, gelatin, ethanol, and the like; and disintegrants such as laminaran, agar, and the like may be exemplified. Furthermore, coloring agents, preservatives, perfumes, flavors, sweetening agents, and other remedies may be contained, when required.

The amount of an FBPase inhibitor contained in the above preparations is not particularly restricted and may be selected from a wide range, but it is usually from 1 to 70 weight percent of the total weight of the preparation, and preferably, the appropriate concentration is from 1 to 30 weight percent.

The dose varies depending on a variety of factors such as the symptoms, age, and body weight of the patient, the formulation, and the like. A suitable dosage level is generally from 0.001 mg (preferably 1 mg, and more preferably 10 mg) per day as a lower limit to 2000 mg (preferably 400 mg, and more preferably 200 mg) per day as an upper limit for a human adult.

The amount of a biguanide preparation contained in the above preparations is not particularly restricted and may be selected from a wide range, but it is usually from 1 to 70 weight percent of the total weight of the preparation, and preferably, the appropriate concentration is from 1 to 30 weight percent.

The dose varies depending on a variety of factors such as the symptoms, age; and body weight of the patient, the formulation, and the like. A suitable dosage level is generally from 0.001 mg (preferably 0.01 mg, and more preferably 0.1 mg) per day as a lower limit to 2550 mg (preferably 850 mg, and more preferably 500 mg) per day as an upper limit for a human adult.

[Advantages of the invention]

According to the present invention, high therapeutic efficacy with remarkable reduction of side effects can be achieved by administration of a pharmaceutical composition including an FBPase inhibitor at a specified timing, and therapeutic agents against diabetes mellitus and their administration procedures of the present invention are extremely useful for the treatment of diabetes mellitus.

Furthermore, high therapeutic efficacy and remarkable reduction of side effects can be achieved by co-administration of an FBPase inhibitor and an anti-diabetic agent having a different mechanism of action from that of the FBPase inhibitor at specified timings suitable for each of them, and therapeutic agents against diabetes mellitus and their administration procedures of the present invention are very useful for the treatment of diabetes mellitus.

[Best mode for carrying out the invention]

The present invention is illustrated in more detail by the following test examples. However, the present invention is not limited to these examples.

[Examples]

Compound A employed in the Test Examples is a compound disclosed in International publication number WO 01/47935 pamphlet and can be manufactured according to methods described in the said publication.

[Test Example 1]

Effects of 2-amino-5-isobutyl-4-{2-[5-(N,N'-bis((S)-1-ethoxycarbonyl)ethyl) phosphonamido]furanyl}thiazole (Compound A) and metformin on improvement of diabetes mellitus by administering these drugs at specified timings suitable for each of them.

Eleven-week old Zucker diabetic fatty (ZDF) rats, that spontaneously developed diabetes mellitus, were grouped into 3 groups (7 rats per group). Feeding was limited to from 17:00 to 9:00, and Compound A and metformin (purchased from Sigma Chemical Co., Ltd.) were repeatedly administered for 6 weeks. Doses of Compound A and metformin were 100-150 mg/kg each once daily, and administered at 9:00 AM (9:00) or 4:00 PM (16:00). In the group administered Compound A at 9:00 AM, metformin was administered at 4:00 PM (AM-Compound A / PM-metformin administered group; Group A), and in the group administered metformin at 9:00 AM, Compound A was administered at 4:00 PM (AM-metformin / PM-Compound A administered group; Group B). The dose of each compound per day in both groups was fixed at the same. Vehicle alone was administered to the remaining group (control group) twice a day.

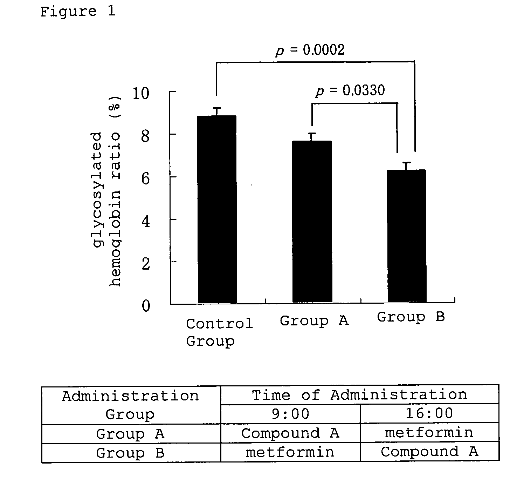

After repeated administration of the compounds for 6 weeks, blood sample was collected from the tail vein of each rat, and ratios of glycosylated hemoglobin levels in erythrocytes were determined by DCA2000 manufactured by Bayer Medical Ltd. Glycosylated hemoglobin is non-enzymatically formed by binding of glucose to hemoglobin, and the ratio of glycosylated hemoglobin is increased by sustained hyperglycemia. Thus the glycosylated hemoglobin ratio is widely used as an indicator of a long-term therapy (or control of blood glucose levels) in patients with diabetes mellitus. The average and standard error of the glycosylated hemoglobin ratio determined in each group were calculated and are shown in Figure 1.

Figure 1 indicates that the glycosylated hemoglobin ratio in Group A was not significantly different from that in the control group (p=0.0749, Tukey test), although the former value was lower than the latter value. On the other hand, the glycosylated hemoglobin ratio in Group B was significantly lower than that in the control group (p=0.0002, Tukey test) and that in Group A (p=0.0330, Tukey test). From these results, it was clearly demonstrated that the administration procedure of "AM-metformin/PM-Compound A Administration" improved diabetes mellitus more remarkably than the other administration procedure of "AM-Compound A/PM-metformin Administration" in rats.

Thus it was revealed that in the co-administration of the said two agents, their improving efficacies against diabetes mellitus were augmented by administration of these drugs at the specified timings, even though the dose level used in each group was at the same level.

Homeostasis of the blood glucose level is maintained by keeping a balance between glucose production in the liver and glucose uptake into various tissues. Gluconeogenesis contributes to the production of glucose in the liver. In general, it is considered that gluconeogenesis in the liver is potentiated during fasting, and glucose uptake into various tissues is relatively superior to gluconeogenesis during feeding.

An FBPase inhibitor suppresses gluconeogenesis by inhibiting FBPase, which are a rate limiting enzyme of gluconeogenesis in the liver. In addition, it is estimated that metformin facilitates glucose uptake into the muscles, as one of its mechanisms of action, although the mechanisms of action of metformin are not fully understood.

Considering these points, the results obtained in the present Test Example 1 are considered to indicate that gluconeogenesis was inhibited by administration of an FBPase inhibitor during the fasting period when gluconeogenesis is active, and glucose uptake into various tissues was facilitated by administration of a biguanide preparation during the feeding period when glucose uptake is active, and consequently, remarkable improvement against diabetes mellitus was obtained by their individual efficient therapeutic effects.

In the present Test Example 1, combined administration of an FBPase inhibitor in the morning and of a biguanide in the evening resulted in the excellent control of blood glucose level, since daytime and nighttime are fasting and feeding periods, respectively, in rats. On the other hand, in the case of humans, since feeding times are generally opposite to those of rats, excellent improving effects against diabetes mellitus are expected to be brought about by combined administration of a biguanide preparation in the morning (preferably before or soon after breakfast) and that of an FBPase inhibitor in the evening (preferably a time in the period from soon after dinner to before bedtime).

From these considerations, combination administration of an FBPase inhibitor and a biguanide preparation at specified timings most suitable for each of them is considered a very excellent therapeutic procedure for patients with diabetes mellitus, because such administration procedures are able to maintain blood glucose levels at favorable levels and to reduce side effects.

[Test Example 2]

Effects of feeding on ameliorating effects of 2-amino-5-isobutyl-4-{2-[5-(N,N'-bis((S)-1-ethoxycarbonyl)ethyl) phosphonamido]furanyl}thiazole (Compound A) against diabetes mellitus

Thirty-six-week old Goto-Kakizaki (GK) rats (purchased from Charles River Japan, Inc.), that spontaneously developed diabetes mellitus, were grouped into 4 groups such as "non-fasting control group", "non-fasting and Compound A administered group", "fasting control group", and "fasting and Compound A administered group" (6 rats per group).

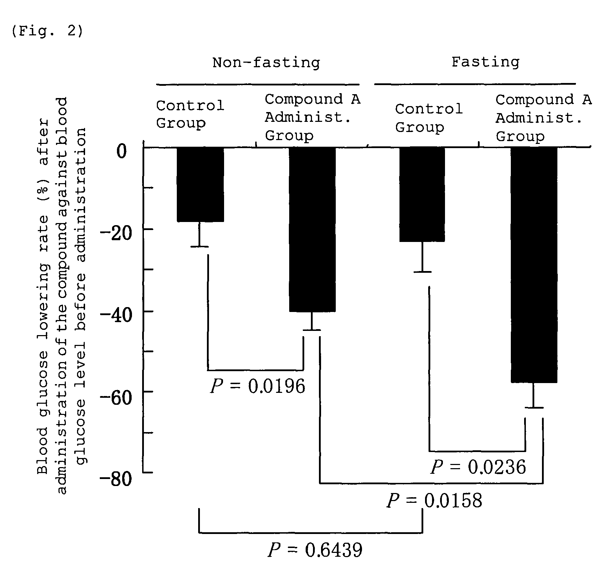

In Compound A administered groups, Compound A (30 mg/kg) was orally administered, and vehicle alone was administered to two control groups. In addition, rats in two fasting groups were fasted from one day before oral administration of Compound A, and rats in two non-fasting groups were fasted immediately after Compound A was administered [the rats were allowed to take food pellets (FR2, manufactured by Funabashi Farm Co., Ltd.) *ad libitum* until fasting was started]. Blood sample was collected from the tail vein immediately before and 4 hours after oral administration of Compound A. Blood glucose level was determined by electric potential measurement method based on glucose oxidase immobilized electrode using Glucoloader GXT (A&T Corp.). The blood glucose lowering rate (%) at 4 hours after administration of Compound A against the blood glucose level before administration was calculated in individual rats according to the following equation, and the average blood glucose lowering rate and its standard error in each group are summarized in Figure 2.

$$[Blood\ glucose\ lowering\ rate(\%)] =$$
$$100 \times ([blood\ glucose\ level\ at\ 4\ hr\ after\ administration] - [blood\ glucose\ level$$
$$before\ administration]) / [blood\ glucose\ level\ before\ administration]$$

As shown in Fig. 2, the blood glucose lowering rate in the non-fasting control group was not significantly different from that in the fasting control group (p=0.6439, t-test), and the blood glucose level in the fasting control group was decreased at 4 hours after vehicle administration by almost the same degree compared with that in the non-fasting control group. However, the blood glucose lowering rate at 4 hours after administration of Compound A was significantly reduced in both fasting and non-fasting groups compared with their corresponding control groups (non-fasting group: p=0.0196, fasting group: p=0.0236, t-test), indicating that Compound A lowered the blood glucose level in both fasting and non-fasting conditions.

On the other hand, comparing the average blood glucose lowering rate of the rats at 4 hours after administration of Compound A in "non-fasting and Compound A administration group" with that in the "fasting and Compound A administration group", the blood glucose lowering rate in the "fasting and Compound A administration group" was significantly lower than that in the "non-fasting and Compound A administration group" (p=0.0158, t-test).

From these results, it was clearly revealed that although Compound A lowered blood glucose levels in both fasted and fed rats, Compound A inhibited blood glucose lowering activity more remarkably when it was administered during a long fasting period.

When these results are applied to human, the longest fasting period (meal interval) in daily life is the period from after dinner to before breakfast. Therefore, it is expected that the improving effects of an FBPase inhibitor are augmented when it is taken from after dinner to bedtime.

Furthermore, based on the results obtained in Test Example 1 and Test Example 2, it is clear that the augmented therapeutic effects obtained by administering Compound A at a suitable time point as described above are not limited only to when Compound A is administered alone, and can be expected when Compound A is administered with other antidiabetic drugs.

[Brief description of the drawings]

Fig. 1 is a graph showing effects of 2-amino-5-isobutyl-4-{2-[5-(N,N'-bis((S)-1-ethoxycarbonyl)ethyl) phosphonamido]furanyl}thiazole (Compound A) and metformin on improvement of diabetes mellitus by administering these drugs at specified timings suitable for each of them (Test Example 1).

Fig. 2 is a graph showing effects of feeding on ameliorating effects of 2-amino-5-isobutyl-4-{2-[5-(N,N'-bis((S)-1-ethoxycarbonyl)ethyl) phosphonamido]furanyl}thiazole (Compound A) against diabetes mellitus (Test Example 2).

**Claims**

1. A pharmaceutical composition comprising an FBPase inhibitor for prophylaxis or treatment of diabetes mellitus, which is **characterized by** augmentation of the prophylactic or therapeutic effects on diabetes mellitus when the pharmaceutical composition is administered at an early time of the longest meal interval.

2. A pharmaceutical composition comprising an FBPase inhibitor for prophylaxis or treatment of diabetes mellitus, which is **characterized by** augmentation of the prophylactic or therapeutic effects on diabetes mellitus when the pharmaceutical composition is orally administered once daily at an early time of the longest meal interval.

3. A pharmaceutical composition according to any one claim selected from Claim 1 or Claim 2, in which the pharmaceutical composition is administered at a certain time in the period from after dinner to bedtime.

4. A pharmaceutical composition according to any one claim selected from Claim 1 or Claim 2, in which the pharmaceutical composition is administered at a certain time range after dinner.

**5.** A pharmaceutical composition according to any one claim selected from Claim 1 to Claim 4, in which the pharmaceutical composition comprises an FBPase inhibitor in an amount of from 1 mg to 400 mg.

**6.** A pharmaceutical composition according to any one claim selected from Claim 1 to Claim 4, in which the pharmaceutical composition comprises an FBPase inhibitor in an amount of from 10 mg to 200 mg.

**7.** A therapeutic procedure for diabetes mellitus, which is **characterized by** once daily oral administration of a pharmaceutical composition comprising an FBPase inhibitor as an active ingredient at an early time of the longest meal interval.

**8.** A kit of a pharmaceutical composition for prophylaxis or treatment of diabetes mellitus, in which at least a single unit of an FBPase inhibitor containing an amount of from 1 mg to 400 mg as a single dose is contained with an indication to take the kit orally once daily at an early time in the period between after-dinner and bedtime.

**9.** A kit of a pharmaceutical composition comprising two different active ingredients to be separately administered at a certain delay for the treatment of diabetes mellitus, said kit containing (a) a pharmaceutical composition comprising a biguanide preparation as the first component of the active ingredients, and (b) a pharmaceutical composition comprising an FBPase inhibitor as the second component of the active ingredients.

**10.** A kit of a pharmaceutical composition comprising two different active ingredients to be separately administered at an appropriate time interval to prevent adverse events in the treatment of diabetes mellitus, which contains (a) a pharmaceutical composition comprising a biguanide preparation as the first component of the active ingredients, and (b) a pharmaceutical composition comprising an FBPase inhibitor as the second component of the active ingredients.

**11.** A kit of a pharmaceutical composition comprising two different active ingredients to be separately administered at an appropriate time interval to prevent adverse events by reducing the dose of a biguanide preparation in the treatment of diabetes mellitus, which contains (a) a pharmaceutical composition comprising a biguanide preparation as the first component of the active ingredients, and (b) a pharmaceutical composition comprising an FBPase inhibitor as the second component of the active ingredients.

**12.** A kit of a pharmaceutical composition according to any one claim selected from Claim 8 to Claim 11, wherein it is clearly indicated that (a) the first component of the active ingredients is to be administered at a time when the patients' glucose uptake into target tissues of the treatment is increased, and (b) the second component of the active ingredients is to be administered when the patients' gluconeogenesis being targeted by the treatment is predominantly facilitated.

**13.** A kit of a pharmaceutical composition according to any one claim selected from Claim 8 to Claim 11, wherein it is clearly indicated that the second component of the active ingredients is to be administered at a time in the period from after dinner to bedtime.

**14.** A kit of a pharmaceutical composition according to any one claim selected from Claim 8 to Claim 11, wherein it is clearly indicated that (a) the first component of the active ingredients is to be administered to the target patients at a time in the period from 4:00 to 12:00, while (b) the second component of the active ingredients is to be administered at a time in the period from 17:00 to 1:00.

**15.** A kit of a pharmaceutical composition according to any one claim selected from Claim 10 to Claim 14, wherein it is indicated that adverse events of the pharmaceutical composition are gastrointestinal disorders.

**16.** A kit of a pharmaceutical composition according to any one claim selected from Claim 10 to Claim 15, wherein it is indicated that an adverse event of the pharmaceutical composition is an increase in lactic acid.

**17.** A kit of a pharmaceutical composition according to any one claim selected from Claim 8 to Claim 16, which is packed for oral administration.

**18.** A kit of a pharmaceutical composition according to any one claim selected from Claim 8 to Claim 17, wherein the biguanide preparation is metformin, phenformin, or buformin.

**19.** A kit of a pharmaceutical composition according to any one claim selected from Claim 8 to Claim 17, wherein the biguanide preparation is metformin.

**20.** A kit of a pharmaceutical composition according to any one claim selected from Claim 8 to Claim 19, wherein the FBPase inhibitor is an agent that inhibits human FBPase activity.

**21.** A kit of a pharmaceutical composition according to any one claim selected from Claim 8 to Claim 19, wherein the FBPase inhibitor is 2-amino-5-isobutyl-4-{2-[5-(N,N'-bis((S)-1-ethoxycarbonyl)ethyl) phosphonamido]furanyl}thiazole or a pharmacologically acceptable salt thereof.

**22.** Use of a biguanide preparation and an FBPase inhibitor for the manufacture of a kit of a pharmaceutical composition comprising two different active ingredients, (a) a biguanide preparation as the first component of the active ingredients and (b) an FBPase inhibitor as the second component of the active ingredients, to be separately administered for the treatment of diabetes mellitus at an appropriate time interval.

**23.** A therapeutic agent for diabetes mellitus, which is **characterized by** separate administration of an agent containing a biguanide preparation as an active ingredient and an agent containing an FBPase inhibitor as an active ingredient at an appropriate time interval.

**24.** A combination of therapeutic agents for diabetes mellitus used in relation to each other, which is **characterized by** administering (a) a pharmaceutical composition comprising a biguanide preparation as an active component and (b) a pharmaceutical composition comprising an FBPase inhibitor as an active ingredient separately at an appropriate time interval.

**25.** A method of treatment for diabetes mellitus, which is **characterized by** separate administration of (a) a pharmaceutical composition comprising a biguanide preparation as an active ingredient, and (b) a pharmaceutical composition comprising an FBPase inhibitor as an active ingredient at an appropriate interval.

**26.** A method of treatment for diabetes mellitus, which is **characterized by** separate administration of (a) a pharmaceutical composition comprising a biguanide preparation as the active ingredient, and (b) a pharmaceutical composition comprising an FBPase inhibitor as the active ingredient at an appropriate interval to reduce adverse events caused by the biguanide preparation.

Figure 1

*p* = 0.0002

*p* = 0.0330

| Administration | Time of Administration | |
|---|---|---|
| Group | 9:00 | 16:00 |
| Group A | Compound A | metformin |
| Group B | metformin | Compound A |

(Fig. 2)

<div align="center">

**INTERNATIONAL SEARCH REPORT**

</div>

| International application No. |
|---|
| PCT/JP2005/022739 |

| A. CLASSIFICATION OF SUBJECT MATTER |
|---|
| *A61K45/00*(2006.01), *A61K31/155*(2006.01), *A61K31/427*(2006.01), *A61P3/10* (2006.01) |

According to International Patent Classification (IPC) or to both national classification and IPC

| B. FIELDS SEARCHED |
|---|
| Minimum documentation searched (classification system followed by classification symbols) |
| A61K31/00-45/08, A61P1/00-3/10 |

Documentation searched other than minimum documentation to the extent that such documents are included in the fields searched

| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
BIOSIS(STN), CAplus(STN), EMBASE(STN), MEDLINE(STN), JCHEM(JOIS), JMEDPlus(JOIS), JSTPlus(JOIS)

| C. DOCUMENTS CONSIDERED TO BE RELEVANT | | |
|---|---|---|
| Category* | Citation of document, with indication, where appropriate, of the relevant passages | Relevant to claim No. |
| X<br><br>Y | JP 2004-508297 A (METABASIS THERAPEUTICS, INC.), 18 March, 2004 (18.03.04), Full text; particularly, Claims; Background of the Invention Par. Nos. [0077], [0079], [0311]; examples<br>& WO 02/03978 A2    & AU 200173271 A<br>& NO 200300034 A    & CZ 200300005 A3<br>& KR 2003031952 A   & SK 200300006 A3<br>& HU 200301830 A2   & BR 200112212 A<br>& EP 1372660 A2     & ZA 200300044 A<br>& NZ 523227 A       & CN 159912 A<br>& MX 2002012713 A1 | 1-6,8-14, 17-20,22-24<br>15,16,21 |

☒ Further documents are listed in the continuation of Box C.   ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 23 February, 2006 (23.02.06) | 07 March, 2006 (07.03.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

## INTERNATIONAL SEARCH REPORT

International application No.

PCT/JP2005/022739

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2004-531463 A (Andrx Labs, LLC.), 14 October, 2004 (14.10.04), Full text<br>& WO 02/36100 A1 & AU 200230830 A<br>& EP 1335708 A1 & KR 2003061392 A<br>& US 6790459 B1 & US 2004/0219209 A1<br>& US 6866866 B1 & NZ 525559 A<br>& MX 2003003994 A1 & US 2006/0008526 A1<br>& US 2006/00008523 A1 & US 2006/0008524 A1<br>& US 2006/0008525 A1 | 15,16,21 |
| Y | Michio HAYASHI, "Biguanide-yaku, separate volume Igaku no Ayumi, Tonyobyo · Taisha Shokogun -state of arts 2004 to 2006, Ishiyaku Shuppan Kabushiki Kaisha, 2004, pages 619 to 620 | 15,16,21 |
| Y | Takaya GOTODA, "2-gata Tonyobyo", Today's Therapy 2004 (Vol.46), Igaku-Shoin Ltd., 2004, pages 492 to 494 | 15,16,21 |
| Y | JP 2003-519154 A (METABASIS THERAPEUTICS, INC.), 17 June, 2003 (17.06.03), Claims; examples<br>& WO 01/47935 A2 & AU 200152447 A<br>& EP 1240174 A2 & NO 200202932 A<br>& BR 200017048 A & US 2002/0173490 A1<br>& KR 2002063248 A & CZ 200202172 A3<br>& SK 200200889 A3 & HU 200204092 A2<br>& CN 1434828 A & ZA 200204399 A<br>& NZ 519219 A & MX 2002006156 A1<br>& US 2005/0004077 A1 & US 6965033 B2 | 21 |
| A | JP 2003-525944 A (METABASIS THERAPEUTICS, INC.), 02 September, 2003 (02.09.03),<br>& WO 01/66553 A2 & AU 200145532 A<br>& US 2002/0040014 A1 & EP 1265907 A2<br>& NO 200204240 A & BR 200109062 A<br>& CZ 200203018 A3 & KR 2002079988 A<br>& SK 200201272 A3 & HU 200300344 A2<br>& ZA 200207004 A & CN 1516705 A<br>& MX 2002008722 A1 & US 6919322 B2<br>& US 2005/0176684 A1 & EP 1607401 A1 | 1-6,8-24 |
| A | JP 2002-524463 A (METABASIS THERAPEUTICS, INC.), 06 August, 2002 (06.08.02),<br>& WO 00/14095 A1 & AU 200010905 A<br>& NO 200101174 A & EP 1112275 A1<br>& CZ 200100840 A3 & BR 9913532 A<br>& SK 200100316 A3 & KR 2001085776 A<br>& CN 1326459 A & HU 200103143 A2<br>& ZA 200101711 A & US 6489476 B1<br>& NZ 510308 A & MX 2001002542 A1<br>& US 2004/0058892 A1 & AU 2003242500 A1 | 1-6,8-24 |

Form PCT/ISA/210 (continuation of second sheet) (April 2005)

**INTERNATIONAL SEARCH REPORT**

International application No.

PCT/JP2005/022739

C (Continuation). DOCUMENTS CONSIDERED TO BE RELEVANT

| Category* | Citation of document, with indication, where appropriate, of the relevant passages | Relevant to claim No. |
|---|---|---|
| A | JP 2003-515523 A (METABASIS THERAPEUTICS, INC.),<br>07 May, 2003 (07.05.03),<br>& WO 00/38666 A2     & AU 200020583 A<br>& NO 200103115 A     & EP 1143955 A2<br>& CZ 200102353 A3    & KR 2001099942 A<br>& BR 9917005 A      & SK 200100917 A3<br>& CN 1350466 A      & ZA 200105016 A<br>& US 6756360 B1     & US 2004/0167178 A1<br>& MX 2001006511 A1   & NZ 512219 A<br>& HU 200402506 A2    & EP 1552850 A2 | 1-6,8-24 |

Form PCT/ISA/210 (continuation of second sheet) (April 2005)

<div align="center">

**INTERNATIONAL SEARCH REPORT**

</div>

| International application No. |
| --- |
| PCT/JP2005/022739 |

**Box No. II      Observations where certain claims were found unsearchable (Continuation of item 2 of first sheet)**

This international search report has not been established in respect of certain claims under Article 17(2)(a) for the following reasons:

1. ☒ Claims Nos.: 7, 25, 26
   because they relate to subject matter not required to be searched by this Authority, namely:
   The inventions as set forth in claims 7, 25 and 26 are relevant to methods for treatment of the human body by therapy.
   (Article 17(2)(a)(i) of the PCT, Rule 39.1(iv) of the Regulations under the PCT)

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

| INTERNATIONAL SEARCH REPORT | International application No. |
|---|---|
| | PCT/JP2005/022739 |

Claims 1 to 6 and 8 to 24

It appears that each of the inventions according to these claims relates to a medicinal composition or a kit having a medicinal composition containing, as the essential components, both of an FBPase inhibitor and a biguanide agent.

These components are each restricted based on the function or property thereof and it is widely known by those skilled in the art that there are compounds having various chemical structures and yet exhibiting the corresponding function or property. However, it is also widely recognized by those skilled in the art that various components so largely differing in chemical structure do not likely exhibit the same effect. To disclose use of which of these components can exert such an effect in a manner sufficiently clear and complete for the clear understanding and embodiment by a person skilled in the art, it is required to confirm the achievement of the same effect by the components having various chemical structures and yet exhibiting the corresponding function or property by a pharmacological test, etc. and specifically show the test data in the description.

In the case of the present application, only use of one example is cited for each of the FBPase inhibitor and the biguanide agent in EXAMPLES and nothing is mentioned concerning use of any other components.

Such being the case, the inventions according to these claims are unclear based on the statements in the claims and the description and these inventions are not disclosed in a manner sufficiently clear and complete for the inventions to be carried out by a person skilled in the art.

Since the inventions the contents of which are not supported as discussed above are described in the present application (PCT Articles 5 and 6), the international search report was made by searching prior art in the scope seemingly reasonable on the basis of the matters mentioned in the description.

Form PCT/ISA/210 (extra sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0014095 A
- WO 0147935 A
- WO 0166553 A
- WO 0203978 A

**Non-patent literature cited in the description**

- *Journal of Medicinal Chemistry,* 2002, vol. 45, 3865-3877
- *Bioorganic & Medicinal Chemistry Letters,* 2001, vol. 11, 17-21